# EUROPÄISCHE PATENTANMELDUNG

(11) **EP 0 678 489 A1**
(43) Veröffentlichungstag der Anmeldung: **25.10.1995**
(21) Anmeldenummer: 94106040.2
(22) Anmeldetag: 19.04.1994
(51) Int. Cl.: C04B 35/111, C04B 35/64

(54) **Alumina-Sinterprodukt und Verfahren zu seiner Herstellung**

(71) Anmelder: FRAUNHOFER-GESELLSCHAFT ZUR FÖRDERUNG DER ANGEWANDTEN FORSCHUNG E.V., D-80636 München (DE)
(72) Erfinder: Krell, Andreas, Dr., D-01217 Dresden (DE); Blank, Paul, Dr., D-01187 Dresden (DE)
(74) Vertreter: Glawe, Delfs, Moll & Partner

(57) **Zusammenfassung**

Die Erfindung bezieht sich auf das Gebiet der Keramik und betrifft ein Al₂O₃-Sinterprodukt und ein Verfahren zu seiner Herstellung, das z.B. als Humanimplantat zur Anwendung kommen kann.

Der Erfindung liegt die Aufgabe zugrunde, hochgradig defektarme Al₂O₃-Sinterprodukte mit hoher Härte anzugeben und herzustellen. Die Aufgabe wird gelöst durch ein Al₂O₃-Sinterprodukt mit 95 bis 100 Vol-% Al₂O₃ und einer Vickers-Kleinlast-Härte ≧ 2000 bei einer Prüflast von 10 N ≦ Prüflast ≦ 100 N (HV1 bis HV10), das ein Gefüge mit einer mittleren Korngröße von ≦ 1,5 µm, mit einer relativen Sinterdichte von ρ ≧ 98,5 % und mit Inhomogenitäten mit einer Häufigkeit von < 5 x 10⁺¹⁰ m⁻² aufweist. Weiterhin wird die Aufgabe gelöst durch ein Verfahren zur Herstellung eines derartigen Al₂O₃-Sinterproduktes, indem aus Ausgangsstoffen ein zunächst ungesintertes äußerst homogenes Vorprodukt mit einer relativen Dichte von ρ ≧ 55 % hergestellt wird, das anschließend getrocknet und gesintert wird.

## Beschreibung

Die Erfindung bezieht sich auf das Gebiet der Keramik und betrifft ein Al₂O₃-Sinterprodukt und ein Verfahren zu seiner Herstellung, das z.B. als Humanimplantat, als Verschleißprodukt, als Schneidwerkzeug oder als Schleifmittel zur Anwendung kommen kann.

Die intensiven Bemühungen der letzten Jahre zur Herstellung monolithischer Al₂O₃-Keramik mit einer Gefügekorngröße unter 2 µm und verbesserten mechanischen Eigenschaften erfolgten im wesentlichen auf zwei Wegen:
- Einsatz von möglichst feinkörnigen Submikrometer- bzw. Nanometer-Pulvern,
- Zusatz von Substanzen zur Senkung der Dichtsintertemperatur.
Als Ausgangssubstanzen werden neben sehr feinkörnigen Al₂O₃-Pulvern auch hochdisperse Übergangstonerden und Al-Oxidhydroxide eingesetzt, letztere generell in Kombination mit die α-Al₂O₃-Bildung fördernden Keimbildnern.

Die sehr feinkörnigen Ausgangssubstanzen haben zwar die erwünschte hohe Sinteraktivität, bringen aber wegen ihres schlechten Verdichtungsverhaltens gleichzeitig erhebliche Probleme bei der Formgebung mit sich.

Das bisher überwiegend angewandte kostengünstige uniaxiale Trockenpressen führt zu unzureichender Gründichte bzw. Dichteinhomogenitäten im Formkörper und bei der Sinterung zu festigkeitsmindernden Defekten.

Aus den genannten Gründen werden andere Formgebungsverfahren, wie kaltisostatisches Pressen, Strangziehen, Druck- bzw. Zentrifugalschlickerguß oder Gelcasting angewandt.

Mittels Strangpressens von keramischen Massen aus Submikrometerpulvern und Sinterung bei 1400 ^{o}C wurde eine Al₂O₃-Gefügekorngröße von 0,8 µm und eine Härte HV20 = 1920 erzielt (Riedel, G. u.a., Silicates Industriels (1989) 1/2,29-35).

Bei sehr feinkörnigen Al₂O₃-Pulvern wird auch der Schlickerguß eingesetzt (Yeh u. Sacks, J. Am. Ceram. Soc. 71(1988),S.841-844), meist in Kombination mit der Druckfiltration (Lange u. Miller, Bull. Am. Ceram. Soc. 66(1987),S.1498-1504) oder einer Vakuum-Druckfiltration (Mizuta u.a., J. Am. Ceram. Soc. 75(1992),S.469-473).

Bei der Verarbeitung kolloidal-disperser Al-Oxidhydroxide oder von Übergangstonerden werden bevorzugt die Sol-Gel-Technik bzw. Gelcasting angewandt. Der Verdichtungsprozeß bei der Sinterung läßt sich durch den Zusatz von Keimen (vorzugsweise α-Al₂O₃) fördern (Kumagai u. Messing, J. Am. Ceram. Soc. 67(1984),S.C230). Ein besonderes Problem der über Sol-Gel-Technik hergestellten Formkörper besteht aber in deren starker Rißanfälligkeit beim Trocknen und Sintern, so daß es äußerst schwierig ist, rißfreie Formkörper im cm-Maßstab und größer herzustellen; selbst mittels Sol-Gel-Technologie erzeugte und gesinterte Schleifkörner einer Größe von nur 0,1 - 1 mm enthalten oft Defekte, z.B. in Form von 0,5 -50 µm großen porösen Gefügebereichen.

Sevinctav u.a. (3.th Euro-Ceramics, Vol.1,1993,S.687-690) nehmen die Formgebung von mit α-Al₂O₃ versetztem Böhmitgel über eine sehr zeitaufwendige Druckfiltration (Filtrationsdauer 12h) vor und erhalten bei einer Sintertemperatur von 1300 ^{o}C rißfreie Scheiben von ca. 2 cm Durchmesser und > 99 % der theoretischen Dichte sowie einer mittleren Gefügekorngröße von 2 µm, ohne jedoch Angaben zur Härte und Festigkeit zu machen.

Heißpressen und heißisostatisches Nachverdichten gesinterter feinstkörniger Al₂O₃- bzw. AlOOH-Pulver bringt meist ein sehr starkes Kornwachstum mit sich (Mizuta u.a., J. Am. Ceram. Soc. 75(1992),S.469-473), so daß Gefüge mit Korngrößen > 2 µm entstehen, was die Erzielung einer hohen Härte verhindert.

Die Möglichkeiten zur Verbesserung der mechanischen Eigenschaften und der Feinkörnigkeit von Al₂O₃-Keramik durch Zusatz von die Sinterung fördernden Substanzen sind sehr beschränkt. Zwar läßt sich so die Dichtsintertemperatur auch von Al₂O₃-Submikrometerpulvern noch deutlich senken (Xue u. Chen, J. Am. Ceram. Soc., 74(1991),S.2011-2013), die erreichte Festigkeit ist jedoch meist gering (um 400 MPa), und die dabei häufig entstehenden Korngrenzenphasen bringen ungünstige Hochtemperatureigenschaften mit sich.

Hinsichtlich der Härte von so hergestellten Al₂O₃-Keramiken ist zunächst der Einfluß der angewandten Prüfbelastung des Indenters zu berücksichtigen. Die Vickers-Härte von Al₂O₃ ist für ein- wie polykristallines Material in komplexer Weise von der Last abhängig (A. Krell, Kristall und Technik 15(1980)12,1467-74).
Typisch ist folgendes Werkstoffverhalten:
- relativ geringer Lasteinfluß bei größerer Prüflast ≧ 50 N,
- sinkende Belastung erzeugt zunächst steigende Härtewerte,
- bei weiter reduzierter Prüflast < 1 bis 5 N sinkt die Härte erneut.

Für die Härtemessung nach Knoop gilt Ähnliches, nur daß zwischen den zahlenmäßigen Ergebnissen bestimmte systematische Abweichungen bestehen.

Nach dem in der Literatur beschriebenen Stand der Technik für die mittels bekannter Verfahren hergestellten Al₂O₃-Keramiken gilt die seit langem gesicherte Erkenntnis, daß unabhängig von der jeweiligen Prüflast eine Korngrößenreduzierung im Bereich von 2 - 0,2 µm keine Härtesteigerung über die bekannten Obergrenzen von HV10 ≈ 2000 (Kleinlasthärte) bzw. HV0,2 ≈ 2500 (Mikrohärte) hinaus ermöglichen kann. Aus den dargelegten Gründen ist dabei ein Vergleich von Härteangaben der Literatur nur dann aussagekräftig, wenn die Lastabhängigkeit der Härte untersucht und die Prüfbedingungen dazu angegeben sind. Dies gilt insbesondere deshalb, weil der Einfluß der Prüflast selbst innerhalb einer Werkstoffgruppe, wie z.B. Sinterkorund, in Abhängigkeit von Dotierungen, Restporosität und vor allem vom Oberflächenzustand extrem unterschiedlich ist. Infolge der vielfältigen und nur selten spezifizierten Angaben im Stand der Technik schwanken die Angaben zum Korngrößeneinfluß auf die Mikrohärte und Kleinlast- oder Makrohärte in weiten Grenzen:
Mikrohärte HV (im Bereich HV0,1-HV0,5, d.h. im Bereich des möglichen Maximums der Härte-Last-Kurve)
- für Einkristalle: 2300 - 2700
   (A.G.Evans u.a.,J.Am.Ceram.Soc. 59(1976)7/8,371-372)
- für Sinterkorund mit D ≈ 2µm
   bei relativer Dichte ρ ≧ 99 %: 2000 - 2600
   (S.D.Skrovanek u.a.,J.Am.Ceram.Soc. 62(1979)3/4,215-216)
- für Sinterkorund-Schleifmittel mit D ≈ 0,20-0,40 µm, mit α-Keimen mittels Sol-Gel-Technik hergestellt, bei relativer Dichte ρ ≈ 98 - 98,5 %: 1900 - 250 (R.Bauer, EP 168606)
Vickers-Kleinlast- und Makrohärte (Prüflast ≧ 10 N)
- für Einkristalle: 1400 - 1700
   (A.Krell, Kristall und Technik 15(1980)12,1467-1476)
- für Sinterkorund mit D ≈ 2 µm
   bei relativer Dichte ρ ≧ 99 %: 1650 - 1850
   (A.Krell, Kristall und Technik 15(1980)12,1467-1476)
- für Sinterkorundprodukte mit D ≈ 0,45 µm, pulvertechnologisch oder auch mittels Sol-Gel-Technik mit α-Keimen hergestellt,
   bei relativer Dichte ρ ≈ 98 -98,5 %: 1900 -2000
   (G.Riedel u.a.,Silicates Industriels (1989)1/2,29-35)
Spezielle Untersuchungen von Skrovanek u.a. an nahezu vollständig dichten heißgepreßten Sinterkorunden mit abgestuft-abnehmender Korngröße zeigten einen Härteanstieg proportional D^{-1/2} nur bis in den Bereich von 3 - 4 µm. Nach allgemeiner Auffassung ist dies durch die zunehmende Behinderung der Versetzungsbewegung in den kleineren Körnern bedingt. Weitere Korngrößenreduzierungen bis 1,7 µm erbrachten jedoch keinen weiteren Anstieg der Härte; die Härte blieb in diesem Bereich kleiner Korngrößen konstant.

Der Nachteil der nach dem Stand der Technik bekannten Al₂O₃-Sinterprodukte besteht darin, daß keine hochgradig defektarmen Al₂O₃-Sinterprodukte mit hoher Härte bekannt und herstellbar sind.

Der Erfindung liegt die Aufgabe zugrunde, hochgradig defektarme Al₂O₃-Sinterprodukte mit hoher Härte anzugeben und herzustellen.

Die Aufgabe wird durch die in den Ansprüchen angegebene Erfindung gelöst.

Das erfindungsgemäße Al₂O₃-Sinterprodukt mit 95 bis 100 Vol-% Al₂O₃ und einer Vickers-Kleinlast-Härte ≧ 2000 bei einer Prüflast von 10 N ≦ Prüflast ≦ 100 N (HV1 bis HV10), weist ein Gefüge mit einer mittleren Korngröße von ≦ 1,5 µm, einer relativen Sinterdichte von ρ ≧ 98,5 % und Inhomogenitäten mit einer Häufigkeit von < 5 x 10⁺¹⁰ m⁻² auf, wobei die Inhomogenitäten Risse und/oder poröse Gebiete entlang der Grenzen von Pulveraggregaten/Pulveragglomeraten und/oder nestartige Gefügebereiche aufgelockerter mit Poren durchsetzter Gefügestruktur und/oder Poren mit einem die doppelte Gefügekorngröße übersteigenden Durchmesser sind.

Bei dem erfindungsgemäßen Verfahren zur Herstellung eines Al₂O₃-Sinterproduktes mit 95 bis 100 Vol-% Al₂O₃ und einer Vickers-Kleinlast-Härte ≧ 2000 bei einer Prüflast von 10 N ≦ Prüflast ≦ 100 N (HV1 bis HV10) wird aus Ausgangsstoffen ein zunächst ungesintertes Vorprodukt hergestellt mit einer relativen Dichte von ρ ≧ 55 % und mit den Verdichtungsprozeß beim Sintern lokal heterogenisierenden Inhomogenitäten nur in Form einzelner voneinander isolierter Defekte mit einer Häufigkeit von < 5 x 10⁺¹⁰m⁻², wobei die Inhomogenitäten Risse und/oder Gebiete erhöhter Porosität entlang der Grenzen von Pulveraggregaten/Pulveragglomeraten sind.

Dieses Vorprodukt wird anschließend getrocknet und gesintert. Vorteilhafterweise wird als Ausgangspulver ein α-Al₂O₃-Pulver mit einer mittleren Korngröße d₅₀ ≦ 0,3 µm und einer chemischen Reinheit von > 99,9 % Al₂O₃ eingesetzt.

Übliche Dotierungen (wie z.B. Magnesium) zur Intensivierung des Verdichtungsprozesses sowie zur Beeinflussung der Gefügeentwicklung beim Sintern können im Rahmen des erfindungsgemäßen Verfahrens zusätzlich zu den in den Ansprüchen 2 und 3 beschriebenen Maßnahmen vorgenommen werden.

Angesichts des umfangreichen bekannten Standes der Technik war es außerordentlich überraschend, daß Sinterprodukte mit einer Vickers-Kleinlast-Härte ≧ 2000 bei einer Prüflast von 10 N ≦ Prüflast ≦ 100 N (HV1 bis HV10) angegeben und erzeugt werden konnten durch das Herstellen einer mittleren Korngröße im Gefüge von ≦ 1,5 µm und einer relativen Sinterdichte von ρ ≧ 98,5 %.

Die Untersuchungen, wie auch der bekannte Stand der Technik haben ergeben, daß allein eine wie erfindungsgemäß genannte relative Sinterdichte in einem Sinterprodukt nicht ausreichend für eine höhere Härte ist. Erst die Kombination einer Gefügekorngröße ≦ 1,5 µm mit einer relativen Sinterdichte ρ ≧ 98,5 % und mit dem Auftreten von maximal 5 x 10⁺¹⁰ m⁻² Inhomogenitäten im ungesinterten Vorprodukt und im fertigen Sinterprodukt führen zu dieser höheren Vickers-Kleinlast-Härte.

Für das erfindungsgemäße Verfahren ist charakteristisch, daß durch die Kombination einer hohen mittleren integralen Dichte des ungesinterten "grünen" Vorproduktes mit einer hohen Homogenität im mikroskopischen lokalen Aufbau eine außerordentliche Senkung der für die erforderliche relative Sinterdichte ≧ 98,5 % notwendigen Sintertemperatur ermöglicht wird. Diese Verbindung von niedriger Sintertemperatur und hoher resultierender Dichte ist die Voraussetzung für die Erzeugung von gleichermaßen hochdichten wie auch feinkörnigen Sintergefügen mit Korngrößen von ≦ 1,5 µm, vorzugsweise sogar ≦ 0,8 µm.

Ein weiterer besonderer Vorteil des erfindungsgemäßen Verfahrens besteht darin, daß es für in der keramischen Industrie verbreitete Technologien nutzbar ist, welche auf diese Weise die Herstellung auch größerer hochgradig defektarmer Formkörper mit hoher Härte ermöglichen.

Die erfindungsgemäßen Sinterkörper können mit den in der keramischen Industrie verbreiteten und vorhandenen Technologien unter Beachtung des erfindungsgemäßen Verfahrens hergestellt werden. Sowohl Schlickertechnologien als auch Sol-Gel-Techniken sind verwendbar.

Im weiteren wird die Erfindung an mehreren Ausführungsbeispielen erläutert. Im Beispiel 1 ist die beste Ausführungsform angegeben.

### Beispiel 1

150 g α-Tonerde Taimicron TM-DAR (d₅₀= 0,19 µm, BET= 14 m²/g) werden mittels eines Rührwerkes in einer wäßrigen Lösung aus 120 ml destilliertem Wasser, 9 ml 10%-ige Polyvinylalkohol-Lösung und 3 ml Glyzerin 30 min dispergiert.
Die Dispersion wird anschließend in einer Labor-Rührwerkskugelmühle mit YTZP-Mahlgarnitur 1 h bei einer Rührerdrehzahl von 1000 U/min gemahlen und homogenisiert und danach einer Gefriertrocknung unterzogen.

Der auf eine Restfeuchte von < 2 % gefriergetrocknete Versatz wird durch ein 300 µm-Sieb gestrichen und bei 30 MPa uniaxial zu einem Formkörper vorverdichtet. Der Formkörper wird anschließend in einer dünnen elastischen Hülle kaltisostatisch bei 700 MPa verpreßt. Die erreichte relative Dichte beträgt 61,0 % der theoretischen Dichte.

Der Formkörper wird bei 80 ^{o}C getrocknet, bei 800 ^{o}C in Luft vorgebrannt (Aufheizrate 0,3 K/min, 1 h Haltezeit bei 800 ^{o}C) und anschließend in Luft bei 1400 ^{o}C drucklos gesintert (Aufheizrate 2 K/min, Haltezeit 2 h bei Sintertemperatur, Ofenabkühlung).

Defekte im Sinterprodukt treten ausschließlich in Form einzelner Poren der Größe 1 -30 µm mit einer geringen Häufigkeit von 8,5 x 10⁺⁷ m⁻² auf.

Die Eigenschaftsuntersuchungen sind an geschliffenen Biegebruchstäben (Diamantschleifscheibe 40/50 µm/Naßschliff/Zustellung 0,01-0,02 mm) aus dem Formkörper durchgeführt worden, was insbesondere für die Ermittlung applikationsbezogener Härtedaten sinnvoll ist, da im technischen Einsatz fast ausschließlich geschliffene Teile verwendet werden.
Im Vergleich sind Härtewerte für polierte Oberflächen angegeben.
Die Dichte ist mittels der Auftriebsmethode bestimmt worden.
Die Korngrößencharakterisierung erfolgte als Linienschnittanalyse an polierten und thermisch geätzten Flächen (Korngröße = 1,56 x mittlere Sehnenlänge).

Folgende Eigenschaftswerte wurden ermittelt:

| | | |
|---|---|---|
| Dichte: | | 3,941 g/cm³ (absolut) 98,9 % (relativ) |
| mittlere Gefügekorngröße: | | 0,65 µm |
| Härte | HV10: | 2258 ± 101 |
| | HV3: | 2514 ± 124 |
| | HV1: | 3055 ± 208 |
| Härte der polierten Oberfläche HV10: | | 2055 ± 94 |
| Bruchfestigkeit: (3-Pkt-Biegung) | | 653 ± 32 MPa. |

Eine Bewertung des Ergebnisses kann durch Gegenüberstellung mit 99 % dichten, durch heißisostatisches Pressen hergestellten geschliffenen Vergleichs-Biegebruchstäben aus Al₂O₃ + 35 Vol-% TiC mit einer mittleren Gefügekorngröße von 1,7 µm erfolgen.
Für diese Vergleichsproben sind folgende Werte ermittelt worden:

| | | |
|---|---|---|
| Härte | HV10: | 2290 ± 76 |
| | HV3: | 2386 ± 173 |
| | HV1: | 2510 ± 182. |

Die erfindungsgemäßen Al₂O₃-Sinterkörper, die durch druckloses Sintern an Luft hergestellt worden sind, zeigen eine Härte, welche das Niveau heißisostatisch gepreßter Komposite mit hohem Hartstoffanteil erreicht und sogar übersteigt.

### Beispiel 2

In 80 ml destilliertem Wasser, das mit 1n HNO₃ auf pH = 5 eingestellt wurde, werden unter Rühren 200 g einer mittels Zentrifugalklassierung aus der α-Tonerde Taimicron TM-DAR gewonnenen Kornfraktion < 0,2 µm (d₅₀ = 0,12 µm) eingebracht, wobei der pH-Wert laufend auf 5 nachgestellt wird.

Die Suspension wird danach noch 1 h mittels Rührwerk und gleichzeitiger Ultraschallbehandlung intensiv dispergiert, über ein 30 µm-Sieb abgesiebt und in einer Druckfiltrationszelle über einem 0,1 µm-Membranfilter bei 3,5,MPa zu Scheiben von 60 mm Durchmesser und 6 mm Dicke verpreßt.

Die Formkörper werden über 3 Tage schrittweise zwischen 20 und 80 ^{o}C auf eine Restfeuchte von 2 bis 3 % getrocknet und danach in elastischen Hüllen bei 700 MPa kaltisostatisch nachverdichtet und nochmals 5 h bei 80 ^{o}C getrocknet, was zu einer relativen Dichte von 60,2 % der theoretischen Dichte führt.

Die Formkörper werden dann bei 800 ^{o}C in Luft vorgebrannt (Aufheizrate 0,3 K/min, 1 h Haltezeit bei 800 ^{o}C) und bei 1350 ^{o}C drucklos gesintert (Aufheizrate 2 K/min, 2 h Haltezeit bei Sintertemperatur, Ofenabkühlung).

Keramographische Untersuchungen zeigten nur vereinzelte kleine Poren der Größe 1 - 5 µm mit einer Häufigkeit von 3 x 10⁺⁹ m⁻².

Die Probenpräparation und -charakterisierung erfolgte wie in Beispiel 1.

Folgende Eigenschaftswerte wurden ermittelt:

| | |
|---|---|
| Dichte: | 3,940 g/cm³ (absolut) 98,8 % (relativ) |
| mittlere Gefügekorngröße: | 0,40 µm |
| Härte HV10: | 2286 ± 74 |

### Beispiel 3

Zu einem Gemisch aus 80 ml destilliertem Wasser, 3,7 ml Dispergierhilfsmittel Dolapix CE64 und 18,5 ml Mg(NO₃)₂-Lösung (enthaltend 1,18 g Mg(NO₃)₂x6H₂O) werden unter Rühren 370 g α-Al₂O₃-Pulver der Sorte Taimicron TM-DAR zugemischt.

Die Suspension wird danach noch 1 h mittels Rührwerk und gleichzeitiger Ultraschallbehandlung intensiv dispergiert, über ein 30 µm-Sieb abgesiebt und in einer Druckfiltrationszelle über einem 0,1 µm-Membranfilter bei 3,5 MPa zu Scheiben von 60 mm Durchmesser und 6 mm Dicke verpreßt.

Die Formkörper werden über 3 Tage schrittweise zwischen 20 und 110 ^{o}C getrocknet, was zu einer relativen Dichte von 61,5 % führt. Die Formkörper werden dann bei 800 ^{o}C in Luft vorgebrannt (Aufheizrate 0,3 K/min, 1 h Haltezeit bei 800 ^{o}C) und bei 1275 ^{o}C drucklos an Luft gesintert (Aufheizrate 2 K/min, 2 h Haltzeit bei Sintertemperatur, Ofenabkühlung).

Die keramographische Analyse des Sinterproduktes zeigt einzelne Poren mit einer Häufigkeit von 2 x 10⁺⁸ m⁻² und zusätzlich nestartige Porenansammlungen mit einer Häufigkeit von 7 x 10⁺⁸ m⁻²; die typische Größe dieser porösen Gebiete beträgt 3 bis 50 µm.

Die Probenpräparation und -charakterisierung erfolgte wie im Beispiel 1.

Es wurden folgende Eigenschaftswerte ermittelt:

| | |
|---|---|
| Dichte: | 3,947 g/cm³ (absolut) 99,0 % (relativ) |
| mittlere Gefügekorngröße: | 0,76 µm |
| Härte HV10: | 2362 ± 85 |
| Härte der polierten Oberfläche: | |
| Härte HV10: | 2105 ± 57 |

## Patentansprüche

1. Al₂O₃-Sinterprodukt mit 95 bis 100 Vol-% Al₂O₃ und einer Vickers-Kleinlast-Härte ≧ 2000 bei einer Prüflast von 10 N ≦ Prüflast ≦ 100 N (HV1 bis HV10), dadurch gekennzeichnet, daß das Sinterprodukt ein Gefüge mit einer mittleren Korngröße von ≦ 1,5 µm, mit einer relativen Sinterdichte von ρ ≧ 98,5 % und mit Inhomogenitäten mit einer Häufigkeit von < 5 x 10⁺¹⁰ m⁻² aufweist, wobei die Inhomogenitäten Risse und/oder poröse Gebiete entlang der Grenzen von Pulveraggregaten/Pulveragglomeraten und/oder nestartige Gefügebereiche aufgelockerter mit Poren durchsetzter Gefügestruktur und/oder Poren mit einem die doppelte Gefügekorngröße übersteigenden Durchmesser sind.

2. Verfahren zur Herstellung eines Al₂O₃-Sinterproduktes mit 95 bis 100 Vol-% Al₂O₃ und einer Vickers-Kleinlast-Härte ≧ 2000 bei einer Prüflast von 10 N ≦ Prüflast ≦ 100 N (HV1 bis HV10), dadurch gekennzeichnet, daß aus Ausgangsstoffen ein zunächst ungesintertes Vorprodukt mit einer relativen Dichte von ρ ≧ 55 % und mit den Verdichtungsprozeß beim Sintern lokal heterogenisierenden Inhomogenitäten nur in Form einzelner voneinander isolierter Defekte mit einer Häufigkeit von < 5 x 10⁺¹⁰m⁻², wobei die Inhomogenitäten Risse und/oder Gebiete erhöhter Porosität entlang der Grenzen von Pulveraggregaten/Pulveragglomeraten sind, hergestellt wird, welches anschließend getrocknet und gesintert wird.

3. Verfahren nach Anspruch 2, dadurch gekennzeichnet, daß als Ausgangspulver ein α-Al₂O₃-Pulver mit einer mittleren Korngröße d₅₀ ≦ 0,3 µm und einer chemischen Reinheit von > 99,9 % Al₂O₃ eingesetzt wird.
